# EUROPEAN PATENT APPLICATION

(11) **EP 4 786 578 A1**
(43) Date of publication of application: **05.08.2026**
(21) Application number: 24871117.8
(22) Date of filing: 26.09.2024
(51) Int. Cl.: C12N 1/20, A01N 63/20, A61K 35/747, A23K 10/18, A23L 33/135, A23L 33/16, C12P 13/00, B09C 1/10, C12R 1/225

(54) **LACTIPLANTIBACILLUS PENTOSUS STRAIN CECT 30896**

(30) Priority: 29.09.2023 ES 202330818
(71) Applicant: Universidad de Jaén, 23071 Jaén (ES)
(72) Inventor: ABRIOUEL HAYANI, Hikmate, 23071 Jaén (ES); BENOMAR EL BAKALI, Nabil, 23071 Jaén (ES); MANETSBERGER, Julia, 23071 Jaén (ES); CABALLERO GÓMEZ, Natacha, 23071 Jaén (ES)
(74) Representative: Clarke, Modet y Cía., S.L.
(86) International application number: PCT/ES2024/070590
(87) International publication number: WO 2025/068625

(57) **Abstract**

The present invention describes a new strain of *L. pentosus* CF-6HA (CECT 30896), isolated from allorean table olives, with dual effect as a probiotic bacterium for humans/animals and also for plants, and its use as an antimicrobial agent and for selenium bioremediation.

## Description

### TECHNICAL FIELD

The present invention relates to the field of Microbiology, in particular, refers to identification of new strains of microorganisms for various uses, including their use as probiotics, for functional compound production, as well as their use in the bioremediation of soils contaminated with metals.

### STATE OF THE PRIOR ART

The growing appearance of multidrug-resistant pathogenic microorganisms (MDR) in humans, animals and plants currently poses a global health challenge, aiming to limit the spread of these pathogens and their antimicrobial/virulent genes through different ecosystems. In this sense, the promotion and increase in the use of probiotics, especially those of plant origin, provide a good opportunity to optimize and balance the health of humans, animals, plants and ecosystems.

Probiotics are defined as live microorganisms that, when administered in adequate amounts, confer a benefit to the host health. They include microorganisms from different sources, such as soil; plants; food; the gastrointestinal, urogenital and vaginal tracts; breast milk and feces of human subjects. They correspond to microorganisms of very different taxonomic groups, which are present in the environment with different bioavailability, and which present a great variety of metabolic activities and different mechanisms of action. The common characteristic that groups them together is their ability to promote health, whether human, animal or plant.

The functional characterization of probiotics depends largely on the specific strain and its origin. In this sense, genomics provides valuable and interesting knowledge about the mechanisms and functionality of probiotics, as well as their health-promoting functions.

Among the probiotics, gram-positive lactic bacteria (BAL) constitute a phylogenetically heterogeneous and versatile group that contains a high probiotic potential, especially the plant-based BAL such as Lactiplantibacillus *pentosus* and *Lactiplantibacillus plantarum* (formerly known as *Lactobacillus pentosus* and *Lactobacillus plantarum*) that are usually isolated from raw and fermented plant foods such as table olives, cucumber, kimchi and pickles, among others. Specifically, allorean table olives, naturally fermented traditional green olives with a protected designation of origin (PDO), are considered a potential source of probiotic strains of *L. pentosus*.

The versatility of *L. pentosus* and *L. plantarum* is due to their genomic diversity, plasticity and functionality linked to their ecological niches that allow them to adapt to the gastrointestinal tract and changing environmental conditions (**Pérez-Díaz et al., 2021**).

Previous studies have shown that *L. pentosus* strains have good capacity for growth and survival in simulated gastrointestinal conditions (acidic pH of 1,5, up to 4% bile salts and 5 mM nitrate), good capacity for self-aggregation and coaggregation with pathogenic bacteria, adherence to intestinal and vaginal cell lines, antimicrobial activity and fermentation of prebiotics and lactose (**Pérez Montoro et al., 2016**). It should be noted that *L. pentosus* strains isolated from Alorean table olives such as *L. pentosus* MP-10 and *L. pentosus* CF2-10N are especially interesting for their genetic diversity and functionality, which gives them great adaptability (**Abriouel et al., 2022**). The *in silico* analysis and some *in vitro* tests of these strains confirmed their safety with respect to their use as probiotic food supplements.

A promising area of research is the search for probiotics that can interact with different hosts and fulfill different functions at the same time.

### DESCRIPTION OF THE INVENTION

Described herein is a probiotic strain with dual effect as a beneficial bacterium for humans and other animals and also for plants, *L. pentosus* CF-6HA, isolated from allorean table olives, which has been deposited with the Spanish Type Culture Collection (CECT) under deposit number CECT 30896.

Although this strain shares several phenotypic and genotypic characteristics with *L. pentosus* strains MP-10 and CF2-10N, isolated from the same ecological niche, there are some characteristics linked to the strain that give each of them a differentiated probiotic potential and functionality (Abriouel **et al., 2012; Pérez Montoro et al., 2016**).

The *in silico* study of its genome confirmed the safety of *L. pentosus* CF-6HA (CECT 30896) due to the absence of virulence determinants and genes of acquired resistance to antibiotics in its genome, and allowed the detection of a wide variety of genes encoding defense and adaptability to different lifestyles, in addition to functional properties, which give the strain its robustness and probiotic potential.

Genes were identified that code for the synthesis of interesting secondary metabolites such as:
- bacteriocins; proteins or peptide toxins synthesized by bacteria that inhibit the growth of similar bacteria or strains close to those that produce them. Some of bacteriocins identified are pediocin, used as a preservative in plant and meat products, and with a high activity against *Listeria* species, and plantaricin Y, characterized by a strong inhibitory activity against *Listeria monocytogenes*;
- various exopolysaccharides;
- terpenes; which exercise different functions in plants, mainly protection against insects and herbivorous animals and protection against high temperatures;
- type III polyketide synthases (T3PKS), with important biological functions and pharmaceutical activities;
- polyketides (PKS); which participate in the biosynthesis of some lipid compounds and several secondary metabolites, and have significant biological functions and important pharmaceutical activities;
- non-ribosomal peptides (NRP), with an extremely wide range of biological and pharmacological activities, which can act as toxins, siderophores or pigments, or have antibiotic, cytostatic or immunosuppressive activity;
- peptide-polyketide (PK+NRP) hybrid products, compounds with antimicrobial and anticancer activity;
- post-translationally modified peptides (RiPP);
- proteins involved in adhesion and other functions.

Especially interesting is the role of this strain as a probiotic agent for humans and animals by maintaining or restoring the host natural microbiota. In addition, both in humans and animals and in plants, it can reduce the spread of pathogens and their resistance genes. It stands out for its antimicrobial activity against human and animal pathogens and plant pests, such as *Pseudomonas syringae* and *Verticillium dahliae*.

The strain of the invention can be used alone or in combination with the aforementioned L. *pentosus* MP-10 and *L. pentosus* CF2-10N.

Finally, *L. pentosus CF-6HA* (CECT 30896) has the ability to biotransform selenite into nanoparticles (SeNPs) so it can be used in selenium bioremediation in natural media and as a starter culture for bioenrichment of foods fermented with selenium.

Therefore, a **first aspect** of the invention describes the *Lactiplantibacillus pentosus* strain CF-6HA deposited with the Spanish Type Culture Collection under accession number CECT 30896, hereinafter "strain of the invention". The invention also relates to the pure bacterial culture, supernatant, extract or bioactive compound isolated from said strain, as well as to the composition comprising said culture, supernatant, extract or bioactive compound.

A **second aspect** of the invention describes the non-therapeutic use of the strain of the invention, the pure bacterial culture and/or the composition comprising the same as a probiotic for plants.

A **third aspect** of the invention describes the non-therapeutic use of the strain of the invention, the pure bacterial culture and/or the composition comprising the same as an antimicrobial agent against *Pseudomonas fragi.* Preferably, this antimicrobial agent is used additionally and simultaneously against one or more of the microorganisms selected from the list consisting of: *Enterococcus faecalis, Bacillus cereus, Staphylococcus aureus, Listeria innoccua, Escherichia coli, Salmonella enterica, Candida albicans, Pseudomonas syringae* and *Verticillium dahliae.*

A **fourth aspect** of the invention is the non-therapeutic use of the supernatant, extract or bioactive compound isolated from the strain of the invention and/or composition comprising the same as antimicrobial compound or composition against *Pseudomonas syringae, Verticillium dahliae, Pseudomonas fragi, Staphylococcus aureus, Bacillus cereus, Escherichia coli* and/ or *Candida albicans*.

A **fifth aspect** of the invention relates to the strain of the invention, the pure bacterial culture and/or the composition comprising the same for use as a probiotic medicament for humans and non-human mammals.

A **sixth aspect** of the invention relates to the strain of the invention, the pure bacterial culture and/or the composition comprising the same for use as an antimicrobial medicament against *Pseudomonas fragi.* Preferably, it is used simultaneously as antimicrobial medicament against one or more of the microorganisms selected from the list consisting of: *Enterococcus faecalis, Bacillus cereus, Staphylococcus aureus, Listeria innoccua, Escherichia coli, Salmonella enterica, Candida albicans, Pseudomonas syringae* and *Verticillium dahliae.*

A **seventh aspect** of the invention relates to supernatant, extract or bioactive compound isolated from the strain of the invention and/or the composition comprising the same for use as an antimicrobial medicament against *Pseudomonas syringae, Verticillium dahliae, Pseudomonas fragi, Staphylococcus aureus, Bacillus cereus, Escherichia coli* and/ or *Candida albicans*.

An **eighth aspect** of the invention relates to the use of the strain of the invention for obtaining pediocin and Plantaricin Y.

A **ninth aspect** of the invention describes the use of the strain of the invention for the bioremediation of media contaminated with selenium.

A **tenth aspect** of the invention describes the use of the strain of the invention for the bioenrichment of foods fermented with selenium.

Throughout the description and claims the word "comprises" and variants thereof are not intended to exclude other technical features, additives, components or steps. For those skilled in the art, other objects, advantages, and features of the invention will be apparent in part from the description and in part from the practice of the invention. The following examples and drawings are provided by way of illustration, and are not intended to be limiting of the present invention.

### BRIEF DESCRIPTION OF THE DRAWINGS

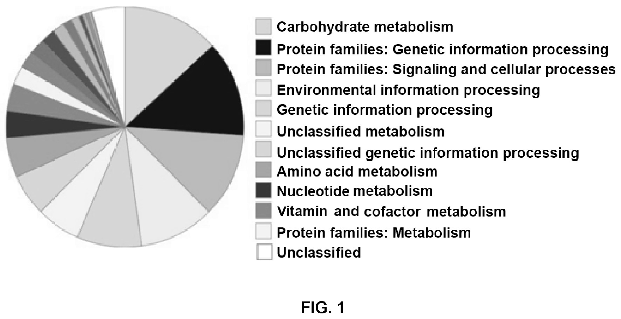

**Figure 1****.** Prediction results made with BlastKOALA of functional categories in the genome of L*actiplantibacillus pentosus* CF-6HA (CECT 30896) and their frequencies.

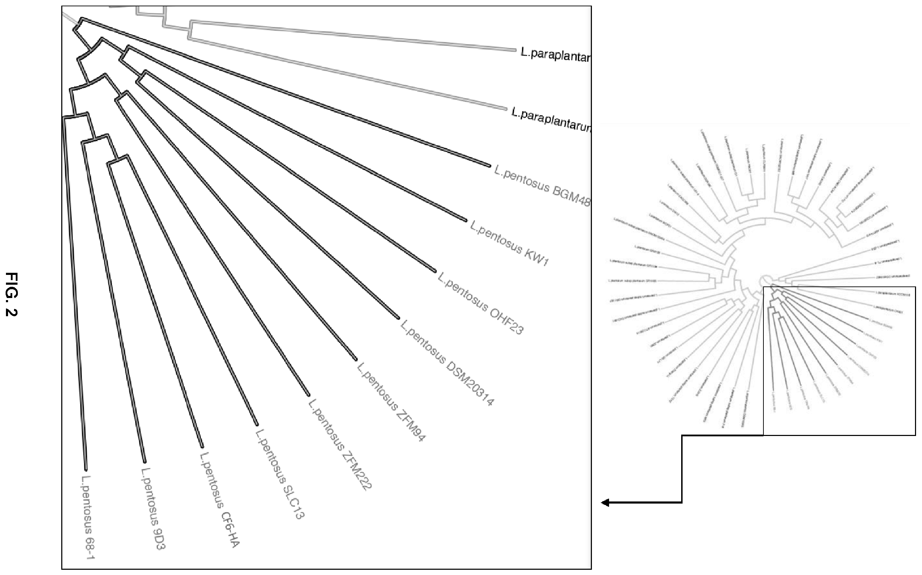

**Figure 2****.** Phylogenetic relationships between genomes of *Lactiplantibacillus* spp. A phylogenetic tree was constructed with ND Tree 1.2 by comparing the genomic sequence of *L. pentosus* CF-6HA (CECT 30896) with the genomic sequences of other *Lactiplantibacillus* spp. (*L. pentosus*, *L. plantarum*, *L. paraplantarum*) that was visualized with the NCBI Tree Viewer.

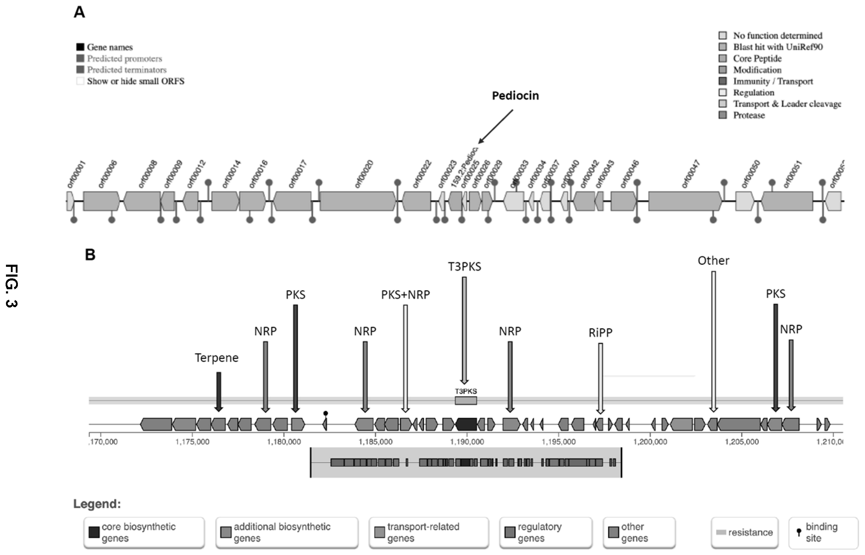

**Figure 3****.** Group organization of predicted antimicrobial biosynthetic genes in genome of *Lactiplantibacillus pentosus* CF-6HA (CECT 30896). **(A)** Predicted bacteriocin gene cluster using BAGEL4 web server. (**B)** T3PKS gene cluster (Type III PKS, region 2.1) predicted using the anti-SMASH software tool. Genes are represented by arrows with different colors that correspond to different functions.

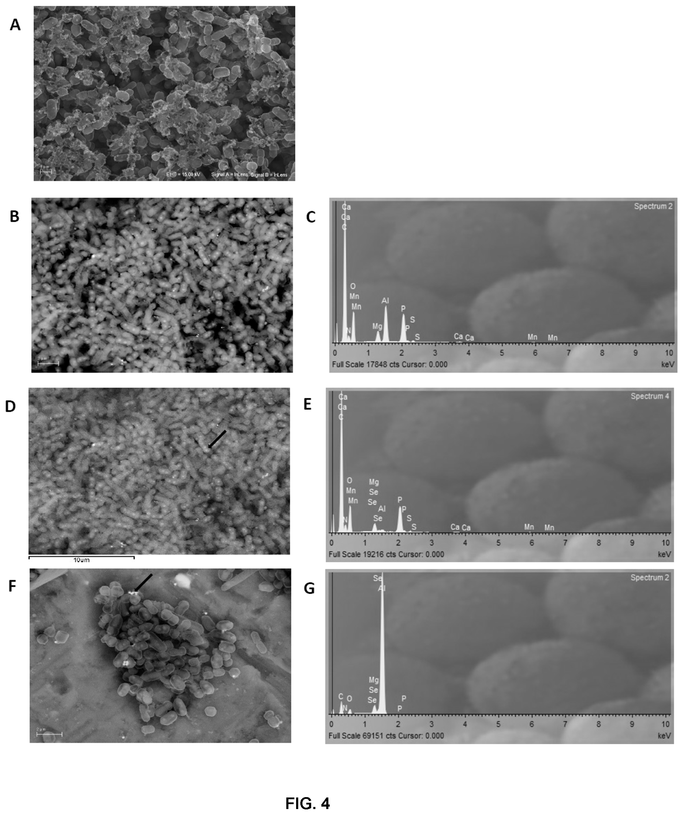

**Figure 4****.** Scanning electron micrographs **(A, B, D and F)** and EDX microanalysis (X-ray energy dispersion microanalysis) **(C, E and G)** of Lactiplantibacillus *pentosus* CF-6HA cells (CECT 30896) cultured in MRS broth (Man, Rogosa and Sharpe) in the absence **(A-C)** and presence of 5 ppm of Se **(D-G)** at 37°C for 24 h. Scanning electron microscope (SEM) images were taken at different magnifications and the arrows indicate the locations of the EDX spectra.

### DETAILED DESCRIPTION OF THE INVENTION

### Bacterial strains and growth conditions

*Lactiplantibacillus pentosus* CF-6HA (deposited with the Spanish Type Culture Collection as CECT 30896), originally isolated from naturally fermented allorean green table olives. *Lactiplantibacillus pentosus* CF-6HA (CECT 30896) was routinely grown at 37°C on Man, Rogosa and Sharpe agar (MRS) under aerobic (atmospheric) conditions for 24-48 h. MRS agar, thanks to its composition, allows the proper development of lactobacilli and other lactic acid bacteria: proteose peptone, meat extract, yeast extract and glucose provide nitrogen, carbon, vitamins and minerals; sorbitan monoleate, sodium, magnesium and manganese salts provide cofactors for bacterial growth and can inhibit development of some microorganisms; ammonium citrate acts as an inhibitory agent for the growth of Gram negative bacteria.

Bacterial and fungal pathogens were obtained from the Spanish Type Culture Collection (CECT), except for *Pseudomonas syringae* strains that were supplied by Dr. J.A. Gutierrez (University of Malaga, Spain). The growth conditions of the pathogens are shown in **Table 1**.

**Table 1: Pathogenic strains and growth conditions used in this study.**

| **Strain** | **Culture medium** | **Growth temperature (°C)** | **Growth time (h)** |
|---|---|---|---|
| *Pseudomonas syringae* subsp. *syringae* UMAF0158 | TSB/TSA | 25 | 24 |
| *Pseudomonas syringae* subsp. *syringae* UMAF0294 | TSB/TSA | 25 | 24 |
| *Pseudomonas fragi* T81 | TSB/TSA | 25 | 24 |
| *Listeria innocua* CECT 910 | TSB/TSA | 37 | 24 |
| *Staphylococcus aureus* CECT 976 | TSB/TSA | 37 | 24 |
| *Staphylococcus aureus* CECT 4465 | TSB/TSA | 37 | 24 |
| *Bacillus cereus* LWL1 | TSB/TSA | 37 | 24 |
| *Bacillus cereus* CECT 148 | TSB/TSA | 37 | 24 |
| *Enterococcus faecalis* S-47 | TSB/TSA | 37 | 24 |
| *Enterococcus faecalis* FI 9190 | TSB/TSA | 37 | 24 |
| *Escherichia coli* CECT 432 | TSB/TSA | 37 | 24 |
| *Salmonella enterica* S62 | TSB/TSA | 37 | 24 |
| *Saccharomyces cerevisiae* CECT 1171 | TSB/TSA | 37 | 24 |
| *Candida albicans* CECT 1001 | MHB/MHA | 26 | 24 |
| *Verticillium dahliae* CECT 2694 | PDA | 28 | 72-120 |
| *Verticillium dahliae* CECT 2884 | PDA | 28 | 72-120 |

| | | | |
|---|---|---|---|
| MHB/MHA: Mueller Hinton Broth/Mueller Hinton Agar; TSB/TSA: Tryptone Soy Broth/Tryptone Soy Agar; PDA: Potato Dextrose Agar. | | | |

All strains were stored in 20% glycerol at -80°C for long-term storage.

### DNA extraction, whole genome sequencing, assembly, annotation and comparative analysis.

The genome sequence of *L. pentosus* CF-6HA (CECT 30896) was sequenced and analyzed as an unequivocal diagnostic tool for its safety (according to the indications of the EFSA panel - Food safety and authorization of regulated products and the panel of FEEDAP-Panel Products or Substances Used in Animal Feed, 2018).

Bacterial cells of *L. pentosus CF-6HA* (CECT 30896), cultured for 18 h at 37°C under aerobic conditions in liquid medium, were harvested by centrifugation and total genomic DNA was extracted using the PureGene core B kit, according to the manufacturer's instructions (Qiagen, Spain) and as described by **Abriouel et al. (2022**).

Library preparation, genome sequencing, assembly, and annotation were carried out as described by Abriouel **et al. (2022).** Briefly, purified genomic DNA was cut into 10 to 20 kb fragments using the protocol designed for the preparation of PacBio RS II system DNA libraries (Pacific Biosciences, Menlo Park, CA) and the resulting libraries (22-24 kb) were purified and sequenced as described by **Abriouel et al. (2022).**

Raw sequence data was filtered (Q20) and a total of 150292 reads were obtained with an average length of 12167 bp. The resulting readings were assembled *de novo* following the Hierarchical Genome Assembly Process (HGAP3.0) method (SMRT analysis version: 2.3.0, patch no. 4) for Pacific Bioscience, as described by **Abriouel et al. (2022**). Next, the prediction of coding DNA sequences (CDS), tRNA, rRNA and mRNA genes and signal peptides was performed on the assembled sequences following the methodologies detailed by **Abriouel et al. (2022).**

Sequence annotation was performed using the BLAST2GO program version 4.1.9 (https://www.blast2go.com/) now part of OmicsBox (https://www.biobam.com/omicsbox/) (**Conesa et al., 2005**), followed by complementary protein domain-specific annotation using the HMMER 3.1 program (July 2017) (http://hmmer.org/) and the database formerly known as Pfam (currently InterPro) (https://www.ebi.ac.uk/interpro/) (Paysan-Lafoss**e et al., 2022**). Blast2GO is a bioinformatics software tool for automatic and high-throughput functional annotation of new sequence data (genes, proteins). It uses the BLAST algorithm to identify similar sequences, and then transfers the existing functional annotation of already characterized sequences to the new sequence. Functional information is represented by Genetic Ontology (GO), an important bioinformatics initiative to unify the representation of the attributes of genes and gene products in all species. On the other hand, HMMER is a program that is used to search for homologous sequences in databases and to perform sequence alignments. It implements methods that use probabilistic models called profile hidden Markov models (profile HMM). HMMER is often used in conjunction with a profile database, such as Pfam or Interpro. Pfam is a database that brings together a wide collection of multiple alignments of sequences and hidden Markov models that covers a large part of protein domains and common families. For each family in Pfam, multiple sequence alignments can be viewed, protein domain architectures and organization examined, species distribution explored, links to other databases followed, and known protein structures viewed.

In addition, a gene blast was performed against protein Clusters of Orthologous Groups (COGs) (http://www.ncbi.nlm.nih.gov/COG) using the WebMGA server (http://weizhonglilab.org/metagenomic-analysis) (**Wu et al., 2011**) and the Kyoto Encyclopedia of Genes and Genomes (KEGG) database. The COGs database is an attempt at phylogenetic classification of proteins encoded in whole genomes. Each COG includes proteins that are inferred to be orthologous (direct evolutionary homologues). The COG database is updated periodically as new genomes become available. COGs can be applied to the task of functional annotation of newly sequenced genomes. On the other hand, WebMGA is a customizable web server for rapid metagenomic analysis that includes more than 20 commonly used tools for sequence grouping, quality control of raw reads, elimination of sequencing artifacts and contaminations, taxonomic analysis, functional annotation, etc. providing rapid analysis of metagenomic data. KEGG is a collection of online databases of genomes, enzymatic pathways, and biological chemicals.

Genome sequencing, assembly, and annotation were performed at Biopolis-ADM (Valencia, Spain). A comparative genomic analysis was performed using the complete genomic sequences available from *Lactiplantibacillus* spp. in the NCBI database and from *L. pentosus* CF-6HA (the strain CECT 30896 object of the present invention). First, the phylogenetic relationship between *L. pentosus* CF2-10N (access number ERR11550479), *L. pentosus* MP-10 (access number GCA_900092635.1), *L. pentosus* DSM 20314 (access number GCA_003641185.1), *L. pentosus* KCA1 (access number GCA_000271445.1) and *L. pentosus* CF-6HA (the strain CECT 30896 object of the present invention) was explored.

Analysis of interspecies synteny was performed by MAUVE alignment (DNASTAR LASERGENE v17.4.2) (**Darling et al., 2004**). Next, a phylogenetic tree was constructed by the maximum likelihood method: RAxML Randomized Axelerated Maximum Likelihood (**Stamatakis, 2014**) using the MAUVE alignment (DNASTAR LASERGENE v17.4.2). The statistical reliability of the tree was evaluated by a bootstrap analysis of 1000 replicates. The bootstrap is a resampling method that is used to approximate the distribution in the sampling of a statistic. It is frequently used to approximate the bias or variance of a statistical analysis, as well as to construct confidence intervals or perform hypothesis contrasts on parameters of interest.

In addition, NDtree 1.2 (**Qian et al., 2003)** was used to construct phylogenetic trees from FASTQ Single-End or Pair-End files of *Lactiplantibacillus* spp. (last accessed March 2023) (**Kaas et al., 2014; Leekitcharoenphon et al., 2014**) and the NCBI Tree Viewer (Tree Viewer 1.19.4 available as an NCBI application) for graphical visualization of phylogenetic trees (https://www.ncbi.nlm.nih.gov/tools/treeviewer/).

The complete genomic sequence of *L. pentosus* CF-6HA (CECT 30896) has a size of 4.148.739 bp and an estimated mol% G+C content of 45,7%. The genomic sequence contains a single circular chromosome of 3.764.590 bp and an estimated mol% G+C content of 46,19% similar to that of its homologous strains such as *L. pentosus* MP-10 (3.698.214 bp; GC content of 46,32%) and *L. pentosus* CF2-10N (3.645.747 bp; GC content of 46,42%) (Abriouel **et al., 2016, 2022**) and 6 plasmids of 38,5-74,5 kb, namely, pLPE6-1 (38.502 bp), pLPE6-2 (73.263 bp), pLPE6-3 (72.520 bp), pLPE6-4 (62.787 bp), pLPE6-5 (74.498 bp) and pLPE6-6 (61.672 bp) unlike the five present in *L. pentosus* MP-10 (29-46,5 kb) and the four in *L. pentosus* CF2-10N (58-120 kb) (Abriouel **et al., 2016, 2022**). These plasmids can play a key role in fermentation process and adaptation to the ecosystem (soil, plant and brine), including heavy metal detoxification (**Abriouel et al., 2019**).

In addition, 16 rRNA genes and 84 tRNA coding sequences have been predicted in the genome of *L. pentosus* CF-6HA (CECT 30896) using mRNAmer (version 1.2). The RNAmmer 1.2 server predicts 5s/8s, 16s/18s, and 23s/28s ribosomal RNA in complete genomic sequences (https://services.healthtech.dtu.dk/services/RNAmmer-1.2/).

Regarding functional classification, the annotated genomic sequence revealed 3.960 open reading frames (ORFs), of which 73,3% (2.902) were attributed to a COG (Cluster of Orthologous Groups) family and/or received a functional description. In addition, the genome of *L. pentosus* CF-6HA (CECT 30896) contains 1.245 KEGG orthology (KO) assignments, mainly enzymes (699 genes) and transporters (212 genes). The frequency of KEGG functional annotations obtained by BlastKOALA (KEGG tool; last updated May 15, 2019; **Kanehisa et al., 2016**), which assigned approximately half (47,4%) of the genes to KEGG annotations corresponding to genetic information processing (248 genes), carbohydrate metabolism (244), cellular signaling and processing (214 genes), environmental information processing (193 genes), genetic information processing (165 genes), unclassified: metabolism (113 genes), unclassified: genetic information processing (106 genes), amino acid metabolism (100 genes), nucleotide metabolism (69 genes), co-factor and vitamin metabolism (68 genes) and metabolism (49 genes) among others (**Figure 1**).

Comparative genomic analysis of *L. pentosus* CF-6HA (CECT 30896) and other *L. pentosus* strains isolated from the same ecological niche (Alorean table olive), such as *L. pentosus* strains MP-10 and CF2-10N, *L. pentosus* DSM 20314 (type strain) isolated from corn silage and *L. pentosus* KCA1 isolated from the vaginal tract, was carried out by sequence alignment using the MAUVE algorithm. The syntenic linkage of *L. pentosus* CF-6HA (CECT 30896) against other *L. pentosus* strains revealed the presence of highly conserved syntenic blocks, although inversions and rearrangements occurred between all *L. pentosus* strains even among those isolated from the same ecological niche (Alorean table olive; *L. pentosus* CF-6HA strains (CECT 30896), MP-10 and CF2-10N). To highlight this relationship, a tree of the central genome of maximum likelihood (RaxML) was constructed and the results revealed that *L. pentosus* CF-6HA (CECT 30896) generally shared 97% identity (evolutionary distance "ED", ED=0,02) with the strains of L. *pentosus* MP-10 (97,62%), CF2-10N (97,62%) and *L. pentosus* DSM 20314 (97,53%), while *L. pentosus* KCA1 only presented 92,16% identity (ED=0,08).

Phylogenetic analysis performed with the NDtree tool, based on the number of nucleotide differences between strains, and the NCBI tree viewer showed a clear distinction between the 48 strains of Lactiplantibacillus *spp.* grouped into strains belonging to the same species (**Figure 2**).

### Genomic analysis of safety and antimicrobial resistance aspects of Lactiplantibacillus pentosus CF-6HA (CECT 30896).

In addition to the global annotation using the BLAST2GO program, the antibiotic resistance genes (ARG) of *L. pentosus* CF-6HA (CECT 30896) were also examined using the Resistance Gene Identifier software (RGI 6.0.1) as part of the CARD tools "The Comprehensive Antibiotic Resistance Database" (https://card.mcmaster.ca/) (**Alcock et al., 2023**), using CARD's cured AMR (antimicrobial resistance) detection models (last accessed in February 2023). RGI is used to predict resistomes from protein or nucleotide data based on homology models and single nucleotide substitutions (SNPs). CARD is a database that collects and organizes reference information on genes, proteins and phenotypes of antimicrobial resistance. The database covers all types of drug classes and resistance mechanisms and structures its data based on an ontology. It allows to find possible antibiotic resistance genes in newly sequenced genomes.

Prediction by BLAST2GO annotation revealed the presence of several non-specific antimicrobial resistance mechanisms that rely on flux transporters or transmembrane proteins involved in drug response, including antibiotics, such as the multidrug transporter MFS (encoded by gene KCA1*_0967*); TIGR00374 family protein (encoded by gene *mprF*); multidrug transporter protein (encoded by genes *FD24_GL001667, LPENT_02485,* and *LPE_*01285); cation effluent protein (encoded by gene *FD24_GL002035*); ABC transporter, ATP-binding protein, and permease (encoded by genes *LPENT_00563*, *LPENT_02775, LPENT_02344* and *LPENT_02438*); ATP-binding cassette transporter permease ABC Superfamily MutG lantibiotic protection (encoded by gene *LPENT_00532*); ATP-binding cassette transporter ABC Superfamily, ABC protein (encoded by gene *LPE_00726*); ATP-binding protein ABC transporter (encoded by genes *LPENT_00338, FD24_GL000723, LPENT_02848, N692_09400, LPE_02094, LPE_00791, FD24_GL003445, FD24_GL002415, LPENT_02562, LPE_01544, LPENT_02495, KCA1_2339, LPENT_02440, gen_2043, LPENT_02320, gen_2108, LPE_01165, FD24_GL000926, FC82_GL001371, FD47_GL001009, LPENT_01461* and *FD24_GL002891*); ABC transporter permease protein (encoded by genes *FD24_GL000827*, *LPENT_02885*, *FD24_GL003057*, and *LPE_01349*); multidrug ABC transporter ATP-binding protein (encoded by *gene_1153*); multidrug ABC transporter permease (encoded by gene *LPE_02342*); putative ABC transporter ATP-binding protein (encoded by gene *LPENT_03065*); integral membrane protein (encoded by genes *LPENT_02708*, *LPENT_02080*, and *LPENT_01044*); ABC-type multidrug transport system, ATPase and permease components (encoded by gene *LPENT_02699*); transporter (encoded by gene *FD24_GL003458*); bacitracin ABC transporter ATP-binding protein (encoded by gene *FD24_GL002522*); DMT superfamily metabolite transporter (encoded by gene *LPENT_02520*); hypothetical protein (encoded by *gene_1979* and *gene_2937*); membrane protein (encoded by gene *KCA1_2377*); EamA family transporter (encoded by gene *LPE_02249*); multicomponent ABC transporter, ATP-binding protein and permease (encoded by gene *LPENT_02375*); ABC transporter (encoded by gene *LPE_03175*); SMR family quaternary ammonium efflux transporter (QacE) (encoded by gene *FD24_GL003284*); MULTISPECIES: quaternary ammonium compound efflux protein (encoded by gene *N692_04340*); transporter protein (encoded by gene *LPE_01191*); MULTISPECIES: undecaprenyl diphosphatase (encoded by gene *uppP*); VanZ family protein (encoded by gene *LPE_02604*); EamA/RhaT family transporter (encoded by gene *KCA1_0065*); MATE family efflux transporter (encoded by gene *LPE_00986*); ATP-binding cassette (ABC) transporter superfamily protein (encoded by gene *LPENT_01629*); ABC transporter-related protein (encoded by genes *LPENT_01583* and *LPENT_01583*); MULTISPECIES: ABC transporter ATP-binding protein (encoded by gene *KCA1_0270*); ABC transporter, ATPase and permease components (encoded by gene *LPENT_01451*); EamA family transporter (encoded by gene *LPENT_01209*); multidrug transporter protein, major facilitator superfamily (MFS) (encoded by gene *KCA1_0714*); GroEL chaperonin (encoded by gene *FD24_GL000282*); and cell surface protein (encoded by *gene_3573* and *gene_3843*). All genes are chromosomally localized except for the *gene_3843* encoding the cell surface protein in plasmid pLPE6-1.

Only one strict match was detected by RGI, which was defined within the similarity limits of the individual AMR detection models and represented probable homologues of AMR genes according to **Alcock et al. (2023**). In this regard, the RGI revealed that the genome of *L. pentosus* CF-6HA (CECT 30896) contained a *vanY* gene in a vanB cluster that conferred resistance to glycopeptides (100% identity with the *vanZ* gene of other strains of *L. pentosus* that was located on the chromosome) and several non-specific antimicrobial resistance mechanisms that depend on flow transporters or transmembrane proteins involved in the response to drugs, including antibiotics.

On the other hand, the search for acquired antibiotic resistance genes/chromosomal mutations mediating antimicrobial resistance in the genome of *L. pentosus* CF-6HA (CECT 30896) was performed using the ResFinder software, an online open resource for the identification of antimicrobial resistance genes in next-generation sequencing data and the prediction of phenotypes from genotypes (https://cge.cbs.dtu.dk/services/ResFinder/) versión 4.1 (**Bortolaia et al., 2020; Camacho et al., 2009; Zankari et al., 2020**), with a % identity (ID) threshold selected from 90% and a minimum length selected from 60% (last accessed in February 2023).

ResFinder did not detect any antimicrobial resistance genes acquired by horizontal gene transfer for aminoglycoside, beta-lactam, colistin, disinfectant, fluoroquinolone, fosfomycin fusidic acid, glycopeptide, MLS series (macrolide, lincosamide, and streptogramine B), nitroimidazole, oxazolidinone, phenicol, pseudomonic acid, rifampicin, sulfonamide, tetracycline, and trimethoprim.

Virulence factor (VF) screening was carried out by annotation of predicted coding regions (CDS) using reciprocal BLAST against the Virulence Factor Database of Bacterial Pathogens (VFDB) (http://www.mgc.ac.cn/VFs/main.htm) considering hits as positive when reciprocal BLAST results were similar and using a sequence similarity cut-off of 80% **(Liu et al., 2019**).

No known virulence factors (VF), including toxins, were identified.

These data confirm the safety status of *L. pentosus* CF-6HA (CECT 30896). They also confirm that an intrinsic antimicrobial resistance is not horizontally transferable, as requested in probiotic strains, although it contributes to defense and fitness against drugs **(Tóth et al., 2021).**

### In silico analysis of safety and antimicrobial resistance of Lactiplantibacillus pentosus CF-6HA (CECT 30896)

Once the safety of *L. pentosus CF-6HA* (CECT 30896) was confirmed, the genome sequence was explored for determinants involved in the robustness and functionality that allow this strain to withstand various stresses in its habitat, during fermentation and also in the gastrointestinal tract.

### Mobiloma analysis

The mobilome (set of mobile genetic elements) of *L. pentosus* CF-6HA (CECT 30896) was analyzed for the presence of conjugative plasmids, transposases, transposons, insertion sequences (IS) and prophage coding genes in the annotated genomic sequence, as detailed by **Abriouel et al. (2022).** To do this, the search for insertion sequences (IS) was performed using the ISfinder search tool (https://isfinder.biotoul.fr/) a database dedicated to bacterial insertion sequences (IS), which is the repository of IS that is indexed together with their DNA sequence and open reading frames or possible coding sequences, the sequence of the element ends and target sites, their origin and distribution, etc**. (Zhang et al., 2004)**.

With regard to IS elements, 17 CDS were detected in the genome of *L. pentosus CF-6HA* (CECT 30896) belonging to nine families and distributed in 12 different bacteria, with IS3 as the most native family of *Acinetobacter baumannii*/*A. lwoffii*.

For the analysis of prophages within the genome of *L. pentosus* CF-6HA (CECT 30896), the version of PHASTER was used (https://phaster.ca/; PHAge Search Tool Enhanced Release, last updated March 2016; corresponding to the updated database of prophages/PHAST viruses "PHAge Search Tool"). PHASTER allows the identification and annotation of prophage sequences in bacterial genomes and plasmids (**Arndt et al., 2016; Zhou et al., 2011**).

In addition, complementary information was obtained from the genomic sequence annotated for the prophage and the transposon/transposase.

As for transposases, the genome of *L. pentosus* CF-6HA (CECT 30896) harbored 98 transposases, of which 31 putative transposases and 67 transposases belong to 10 IS families (2 IS3, 1 IS4/IS5, 8 IS5, 23 IS30, 25 ISL3, 1 IS200/IS605, 2 IS256, 1 IS1380, 2 Mutator family, 2 mulE superfamily). Transposases from the ISL3 and IS30 families were the most prevalent. These transposases were equally distributed on both chromosomes (50 CDs) and plasmids (48 CDS in pLPE6-3, pLPE6-4, pLPE6-5 and pLPE6-6), mainly in pLPE6-6, and appeared as multiple copies.

Prophage search results show the presence of 13 temperate phage regions within the genome of *L. pentosus CF-6HA* (CECT 30896) using the PHASTER bioinformatics tool. Seven of them were intact (Regions 1, 2, 3, 7, 8, 9, and 13; score > 90), one was doubtful (Region 12; score 70±90), and the other five were incomplete (Regions 4, 5, 6, 10, and 11; score < 70). Intact prophage regions of *L. pentosus* CF-6HA (CECT 30896) were mainly related to Lactob_Sha1_NC_019489 (Regions 1, 2, 7, 8 and 9; GC content, 30,30-42,12%; region length, 27,1-46 kb), while incomplete prophage regions were associated with different phages such as Lactob_Sha1_NC_019489 (Region 6, 17,2 Kb), Lactob_phig1e_NC_004305 (Region 4, 15,7 Kb), Erwini_pEp_SNUABM_01_NC_048807 (Region 10, 12 Kb), Photob_PDCC_1_NC_048821 (Region 5, 7,6 Kb) and Paenib_Tripp_NC_028930 (Region 11, 5,5 Kb). We determined that Lactob_Sha_1 showed the highest protein match among the identified prophages. With regard to the questionable regions of the prophages, we only identified one that showed similarity with Staphy_SPbeta_like_NC_029119 (Region 12, 4,9 Kb). Only five prophage regions have attL/attR and integrase sequences, such as regions 1, 2, 4, 6, and 7 located on the bacterial chromosome, and identified in intact and incomplete regions.

### CRISPR

On the other hand, the genes encoding CRISPR (Clustered Regularly Interspaced Short Palindromic Repeats) were examined in the annotated genomic sequence of *L. pentosus* CF-6HA (CECT 30896) and the target location of the CRISPR RNAs was determined using the CRISPRDetect program version 2.4 (http://crispr.otago.ac.nz/CRISPRDetect/predict_crispr_array.html) (**Biswas et al., 2016**).

In the genome of *L. pentosus* CF-6HA (CECT 30896), CRISPR systems I and II (both genes characteristic of type I "*cas3*" and type II "*cas9*" *systems)* were identified as defense mechanisms against mobile genetic elements (i.e., viruses, transposable elements and conjugative plasmids. In this sense, 12 genes were detected, five of them exclusive to *L. pentosus* CF-6HA (CECT 30896). These are responsible for the synthesis of CRISPR-associated proteins, and are organized into two operons (CRISPR-I and CRISPR-II). Regarding CRISPR sets (CR), we identified three unquestionable CRISPR sets using the CRISPRDetect program, located dispersedly on the chromosome (678102-2943655).

With regard to the lifestyle adaptation of *L. pentosus* CF-6HA (CECT 30896) to different stresses, *in silico* exploration of the genome sequence shows the frequent genetic diversification inferred by the mobilome (17 ISI elements, 98 transposases, 13 temperate phage regions) and CRISPR elements (CRISPR systems I and II). The high number of mobile genetic elements in the genome of *L. pentosus* CF-6HA (CECT 30896) is comparable to that of other *L. pentosus* strains also isolated from Alorean table olives, i.e., *L. pentosus* MP-10 (29 transposases, 5 temperate phage regions) and *L. pentosus* CF2-10N (66 transposases, 45 IS elements and 8 temperate phage regions**) (Abriouel et al., 2012, 2017, 2022).** However, the number far exceeded other *L. pentosus* strains isolated from other ecological niches, such as *L. pentosus* IG1 (Spanish-style green olive fermentations; 5 genes encoding transposases), *L. pentosus* KCA1 (vagina; 25 genes encoding transposases) and *L. pentosus* DSM 20314 (corn silage; 14 genes encoding transposases) (Abriouel **et al., 2017**). These characteristics indicated that *L. pentosus* strains isolated from Alorean table olives have a large number of elements, specific to the strain in question, with adaptive function that play an important role in their habitat through chromosomal rearrangements, with special attention to *L. pentosus* CF-6HA (CECT 30896).

In addition, other selective advantages for the survival of *L. pentosus* CF-6HA (CECT 30896) are conferred by 13 prophage regions detected in its genome, seven of which were intact and are mainly prophages of lactobacilli, although the other doubtful and incomplete prophages were related to other bacteria (*Erwinia*, *Photobacterium*, *Paenibacillus* or *Staphylococcus*). In fact, *L. pentosus* CF-6HA (CECT 30896) has the machinery capable of coping with the risk of phage infection in different ecosystems such as some lactobacilli (**Sun et al., 2015**).

The CRISPR system (CRISPR-I and CRISPR-II) as another defense mechanism was also detected in the genome of *L. pentosus* CF-6HA (CECT 30896). In particular, 12 genes that provide protection against mobile genetic elements were detected similarly to *L. pentosus CF2-10N* and *L. pentosus* MP-10. However, both genes and their organization were strain-specific, suggesting that adaptation and fitness may depend on the specific strain (Abriouel **et al., 2017, 2022**).

### Probiotic functionalities of Lactiplantibacillus pentosus CF-6HA (CECT 30896)

### In silico analysis of antimicrobial activity

The annotated genomic sequence of *L. pentosus* CF-6HA (CECT 30896) was analyzed for the presence of genes encoding antimicrobial proteins/peptides and genes encoding bacteriocin production were examined using the BAGEL 4 web server (http://bagel4.molgenrug.nl/), which allows bacterial genomic (meta) DNA to be extracted for bacteriocins and ribosomally synthesized and post-translationally modified peptides (RiPPs), by having databases and a BLAST against the main peptide databases (**van Heel et al., 2018**).

Next, each member of the predicted gene clusters was confirmed by Blastp (https://blast.ncbi.nlm.nih.gov/Blast.cgi) which finds regions of similarity between biological sequences, comparing nucleotide or protein sequences with sequence databases and calculating statistical significance.

In addition, antiSMASH version 7.0 (https://antismash.secondarymetabolites.org/#!/start) (**Blin et al., 2021**) was used to search for clusters of secondary metabolite genes. antiSMASH allows rapid identification, annotation, and analysis of clusters of secondary metabolite biosynthesis genes in bacterial genomes. It integrates and crosses with a large number of *in silico* analysis tools for secondary metabolites that have been previously published.

Both BAGEL and antiSMASH were used for the detection of open reading frames (ORFs) involved in the production of antimicrobial compounds.

RiPPs and bacteriocins (unmodified) were compared in the genome of *L. pentosus* CF-6HA (CECT 30896) with the BAGEL database (BAGEL4). The results obtained showed the gene cluster responsible for bacteriocin biosynthesis and consists of 26 genes with a total length of approximately 20 kb located on the chromosome (**Figure 3A**). In this gene cluster, only one biosynthetic bacteriocin gene encoding the pediocin, class IIa bacteriocin (core peptide, pediocin; location 2.511.611-2.511.967 bp) was detected (**Figure 3A**), however, no pediocin immunity/transport genes were identified although several genes encoding other transporters or resistance genes such as the MFS transporter, the cadmium transporter ATPase of the P family or the transcriptional regulator of the MerR family were detected.

In addition, *in silico* analysis of the genome of *L. pentosus* CF-6HA (CECT 30896) by the BLAST2GO program showed the presence of a putative gene (*LPENT_01393*) that encodes the biosynthesis of Plantaricin Y, class IId bacteriocin (PlnY; location 3.248.967-3.249.260 bp).

On the other hand, genome analysis using the anti-SMASH software tool showed the presence of genes involved in the synthesis of secondary metabolites with antimicrobial potential related to T3PKS (type III polyketide synthase) (**Figure 3B**). In this sense, a 2.1 region (location:1.169.377-1.210.546 nt): T3PKS was identified within the chromosome. This cluster was represented by a core biosynthetic gene (ctg2*_1084*) encoding hydroxymethylglutaryl-CoA synthase; six additional biosynthetic genes (ctg*2_1067, ctg2_1074, ctg2_1076, ctg2_1079, ctg2_1087*, and ctg2*_1105*) encoding peptidase_M50, SMCOG1115:HAD superfamily hydrolase, SMCOG1072 dehydrogenase, SMCOG1141:acyltransferase, SMCOG1123: polyprenol-monophosphomannose synthase ppm1, and SMCOG1001: short chain dehydrogenase/reductase SDR, respectively; a transporter gene (ctg2*_1100*) encoding an MFS transporter and other genes (**Figure 3B**). The results of AntiSMASH blastp indicate the closest compound from the MiBIG database and showed the best matches corresponding to terpene, type III polyketide synthase (T3PKS), polyketides (PKS), non-ribosomal peptide (NRP), peptide-polyketide hybrid products (PK+NRP), post-translationally modified peptide (RiPP) and others, with NRP and PKS being the most abundant classes of biosynthetic gene clusters (**Figure 3B****)**.

The interest of these secondary metabolites, including bacteriocins and exopolysaccharides, was based on their diverse structure and specific bioactivities that enhance the promising probiotic potential of *L. pentosus CF-6HA* (CECT 30896) as a source of antimicrobial and bioactive compounds.

Of note is the ability of some lactic bacteria to produce a wide range of beneficial secondary metabolites such as bacteriocins, diacetyl, acetoin, exopolysaccharides, vitamins (group B), cyclic dipeptides, mevalonic acid, mevalonolactone and butanediol, among others, with antimicrobial activity, antioxidant, host interaction, immunomodulation, etc. (**Crowley et al., 2013; Mora-Villalobos et al., 2020**). However, only a few strains are capable of producing secondary metabolites, while only some of them are externally secreted or produced in low concentration.

### In vitro analysis of antimicrobial activity

In order to complement the results obtained by *in silico* analysis, antimicrobial activity against the pathogens listed in **Table 1**, including phytopathogens (responsible for plague), was examined in *vitro*. The expression of genes encoding bacteriocins in *L. pentosus* CF-6HA (CECT 30896) was thus evaluated to confirm the presence of bacteriocins in the cell-free supernatant and their antimicrobial activity against various pathogens.

To do this, 10 µl of an overnight culture of *L. pentosus* CF-6HA (CECT 30896) was spread on MRS agar and incubated for 24 h; then, the plates were covered with buffered soft Heart-Brain Infusion Agar (SF-BHA; at pH 7,0, 0,1 M) inoculated with indicator strains (**Table 1**) cultured for 24 h.

In addition, the supernatant produced by *L. pentosus* CF-6HA (CECT 30896) was tested for antimicrobial activity in MRS broth using diffusion into agar wells as described above. Antimicrobial activity was assessed by measuring the diameter of the inhibition zones from the edge of the well. In the case of *V. dahliae*, the antifungal activity of *L. pentosus* CF-6HA (CECT 30896) was performed on potato dextrose agar (PDA) plates according to **Mohamad et al. (2018)** evaluating growth inhibition by the following formula: 100×[C-T]/C, where, C is the radial growth of the fungus in the control and T is the same in the dual culture (**Aeron et al., 2011**).

The results of the antimicrobial activity of *L. pentosus* CF-6HA (CECT 30896) against phytopathogens were promising (**Table 2**).

**Table 2: Antimicrobial activity of Lactiplantibacillus pentosus CF-6HA (CECT 30896) against phytopathogens and other pathogens.**

| **Indicator strain** | **Antimicrobial activity of *L. pentosus* (CECT 30896) against indicator strain (inhibition zone)** | |
|---|---|---|
| | **Bacteria** | **Cell-free supernatant** |
| *Pseudomonas syringae* subsp. *syringae* UMAF0158 | + | 12 mm |
| *Pseudomonas syringae* subsp. *syringae* UMAF0291 | + | 12 mm |
| *Pseudomonas fragi* T81 | +++ | 12 mm |
| *Listeria innocua* CECT | +++ | weak |
| *Staphylococcus aureus* CECT 976 | +++ | 10 mm |
| *Staphylococcus aureus* CECT 4465 | +++ | 10 mm |
| *Bacillus cereus* LWL1 | +++ | 14 mm |
| *Bacillus cereus* CECT 148 | +++ | 11 mm |
| *Enterococcus faecalis* S-47 | +++ | weak |
| *Enterococcus faecalis* FI 9190 | ++ | weak |
| *Escherichia coli* CECT 432 | +++ | 10 mm |
| *Salmonella enterica* S62 | +++ | weak |
| *Saccharomyces cerevisiae* CECT 1171 | - | - |
| *Candida albicans* CECT 1001 | + | 9 mm |
| *Verticillium dahliae* CECT 2694 | 36%* | 8%* |
| *Verticillium dahliae* CECT 2884 | 9%* | 0%* |

| | | |
|---|---|---|
| (+), the diameter of the inhibition zone from the growth edge is < 10 mm. (++), the diameter of the inhibition zone from the growth edge is in the range of 10-13 mm. (+++), the diameter of the inhibition zone from the growth edge is > 14 mm. * % growth inhibition according to **Aeron et al. (2011).** | | |

In this sense, the strains *P. syringae* subsp. *syringae* UMAF0291 and UMAF0158 and the strains *Verticillium dahliae* CECT 2694 and CECT 2884 were sensitive to the antimicrobial substances produced *ex-situ* by *L. pentosus* CF-6HA (CECT 30896), since the supernatant of this strain was active against bacterial and fungal phytopathogens. In the CECT 2884 strain of *Verticillium dahliae*, this effect is not observed, possibly because it will require greater amounts of the antimicrobial that is present in the supernatant than the CECT 2694 strain of this same species.

In addition, other pathogens were also sensitive to antimicrobial substances produced by *L. pentosus* CF-6HA (CECT 30896), such as the decomposition bacterium *P. fragi* T81 and other human/animal pathogens, i.e., *Bacillus cereus* strains, *Staphylococcus aureus strains, Enterococcus faecalis* strains, *Listeria innocua*, *Escherichia coli*, *Salmonella enterica* and *Candida albicans*, however no activity was detected against *Saccharomyces cerevisiae* CECT 1171. Although *P. fragi* is not a human pathogen, it represents a reservoir of antibiotic resistance genes that can be transferred to other pathogenic bacteria, thus increasing their danger. Therefore, it is a food chain alteration agent and also a reservoir of antibiotic resistance genes according to Elbehiry et al. (2022).

This antimicrobial activity allows this strain to successfully overcome undesirable and pathogenic microbial species in its habitat and also in the intestine.

### Search for other probiotic properties

To determine the probiotic characteristics related to adhesins, exopolysaccharides, enzymes, antioxidants and selenium metabolism, the annotated genomic sequence of *L. pentosus* CF-6HA (CECT 30896) was examined for the corresponding coding genes.

*In silico* analysis of the annotated genomic sequence of *L. pentosus CF-6HA* (CECT 30896) revealed the presence of an arsenal of genes that encode proteins related to probiotic functions.

Some of these proteins are multifunctional, also involved in other functions such as survival, competitive exclusion of the pathogen to epithelial cells, stress response, drug efflux and others.

Although **Abriouel et al.** (**2016, 2017, 2022**) reported that *L. pentosus* strains MP-10 and CF2-10N have several multi-employed proteins in their genome responsible for their functionality, they are not the same proteins and in cases where they are the same protein, the functions performed are different in each strain and depending on the environmental conditions.

With regard to proteins involved in adhesion, 20 genes encoding adhesion-related proteins were identified, including: 7 cell surface proteins; 4 mucin-binding proteins; 1 adhesion protein; 1 metal ABC transporter permease; 1 manganese ABC transporter substrate-binding protein; 1 ABC transporter ATP-binding protein and permease; 1 ATP-binding cassette (ABC) transporter superfamily substrate-binding protein; 1 metal ABC transporter substrate-binding protein; 1 beta-fructosidase; and 2 hypothetical proteins

Regarding exopolysaccharide biosynthesis, it was associated with four coding genes.

With regard to enzymes involved in key probiotic properties (such as oxidoreductases, transferases, hydrolases, lyases, isomerases, ligases, and translocases), the presence of 1 gene encoding bile salt hydrolase, 2 genes encoding tannase, 1 gene encoding alpha-amylase, 1 gene encoding amylopullulanase, 1 gene encoding catalase, and 23 genes encoding different lactases (beta-galactosidases or beta-glucosidases) was identified.

With regard to antioxidants, 11 genes encoding proteins involved in glutathione biosynthesis, 4 genes encoding 4-carboxymuconolactone decarboxylase, 1 gene encoding 2-Cys peroxiredoxin, 1 gene encoding NAD(FAD)-dependent dehydrogenase, 1 gene encoding putative iron-dependent peroxidase, and 1 gene encoding haloperoxidase were identified.

Finally, the genome of *L. pentosus CF-6HA* (CECT 30896) was examined for genes involved in selenium metabolism and 4 coding genes were identified: serine-tRNA ligase, homocysteine methyltransferase, cysteine sulfinate desulphinase and MULTISPECIES: serine-tRNA ligase.

### In vitro analysis of ability to grow and biotransform selenite

In addition, the ability to grow and biotransform selenite (Na₂ SeO₃*)* of *L. pentosus* CF-6HA (CECT 30896) was tested in *vitro* by spectrophotometrically measuring the residual Se in the supernatant after 24 h of growth in MRS broth supplemented with Se.

The cells were cultured in 5 ml of MRS or MRS-Se broth supplemented with 20 ppm of Se (as Na₂ SeO₃) at 37°C for 24 h as described by **Martínez et al. (2020).** The concentration of SeO²⁻₃ remaining in the supernatants was determined spectrophotometrically by the modified method of Brown and Watkinson (**Kessi et al., 1999**) using a microplate assay **(Martínez et al., 2020).** The results obtained demonstrated its growth capacity, resulting in 100% removal of Se, as no detectable Se was observed.

On the other hand, the selenium biosorption capacity on the surface of *L. pentosus CF-6HA* (CECT 30896) in the form of nanoparticles (SeNPs) was examined by scanning electron microscope (SEM) coupled to X-ray energy dispersion spectroscopy in the presence and absence of Se in culture broth.

To do this, *L. pentosus* CF-6HA (CECT 30896) was cultured in MRS or MRS-Se broth supplemented with 5 ppm of Se (as Na₂ SeO₃) at 37°C for 24 h (**Martínez et al., 2020**). A drop of the bacterial sediment was placed onto SEM sample stubs (ANAME, Spain), dried, and subsequently dehydrated through a graded ethanol series (20%, 40%, 60%, 80%, and 100%; 15 min each) prior to immersion in acetone for 1 h. Thereafter, the stubs were subjected to critical point drying before SEM imaging (FESEM, MERLIN, Carl Zeiss, Oxford) using an energy-dispersive X-ray analyzer (EDX). The presence of Se on the cell surface of *L. pentosus* CF-6HA (CECT 30896) in the form of white nanoparticles (SeNPs) was visualized and confirmed (**Figure 4**). In addition, no transformation of the cell form was observed after growth in the presence of Se and subsequent formation of SeNPs.

This strain tolerates selenium in the culture broth and metabolizes selenium according to the presence of relevant coding genes (serine-tRNA ligase, homocysteine methyltransferase, cysteine sulfinate desulphinase and multispecies: serine-tRNA ligase) for proteins capable of biotransforming and reducing toxic selenite (SeO₃²⁻) into elemental selenium (Se⁰). Thus, the strain can completely remove Se from the culture supernatant.

Other lactobacilli belonging to other species isolated from plants or from other fermented foods (sauerkraut) were able to reduce selenite, such as *L. plantarum* CRL 2030 (**Martínez et al., 2020**) and *L. casei* ATCC 393 (**Qiao et al., 2023**).

This important and effective approach for the bioremediation of selenium contamination by *L. pentosus CF-6HA* (CECT 30896) presents an additional attraction that could be exploited in food, agriculture and the environment where the accumulation of this highly soluble and bioavailable micronutrient can be a great challenge. On the other hand, as a starter culture, *L. pentosus* CF-6HA (CECT 30896) could also be used for the bioenrichment of foods fermented with selenium.

### Statistical analysis

All analyses were performed in triplicate. Statistical analyses were performed with Excel 2016 (Microsoft Corporation, Redmond, WA, USA) to determine means and standard deviations. The bioinformatic analysis was carried out with the R package.

### References

Abriouel et al., 2012. Characterization of lactic acid bacteria from naturally-fermented Manzanilla Aloreña green table olives. Food Microbiol. 32, 308-316. https://doi.org/10.1016/j.fm.2012.07.006
Abriouel et al., 2016. Complete genome sequence of a potential probiotic, Lactobacillus pentosus MP-10, isolated from fermented Aloreña table olives. Genome Announc. 4, e00854-e00816. https://doi.org/10.1128/genomea.00854-16
Abriouel et al., 2017. Insight into potential probiotic markers predicted in Lactobacillus pentosus MP-10 genome sequence. Front. Microbiol. 8, 891. https://doi.org/10.3389/fmicb.2017.00891
Abriouel et al. 2019. New insights into the role of plasmids from probiotic Lactobacillus pentosus MP-10 in Aloreña table olive brine fermentation. Sci. Rep. 9, 10938. https://doi.org/10.1038/s41598-019-47384-1
Abriouel et al., 2022. In silico genomic analysis of the potential probiotic Lactiplantibacillus pentosus CF2-10N reveals promising beneficial effects with health promoting properties. Front. Microbiol. 13, 989824. https://doi.org/10.3389/fmicb.2022.989824
Aeron et al., 2011. Multifarious activity of bioformulated Pseudomonas fluorescens PS1 and biocontrol of Sclerotinia sclerotiorum in Indian rapeseed (Brassica campestris L.). Eur. J. Plant Pathol. 131, 81-93. https://doi.org/10.1007/s10658-011-9789-z
Alcock et al., 2023. CARD 2023: expanded curation, support for machine learning, and resistome prediction at the Comprehensive Antibiotic Resistance Database. Nucleic Acids Res. 51(D1), D690-D699. https://doi.org/10.1093/nar/gkac920
Arndt et al., 2016. PHASTER: a better, faster version of the PHAST phage search tool. Nucleic Acids Res. 44, W16-W21. https://doi.org/10.1093/nar/gkw387
Darling et al. 2004. Mauve: multiple alignment of conserved genomic sequence with rearrangements. Genome Res. 2004 Jul;14(7):1394-403. doi: 10.1101/gr.2289704 https://doi.org/10.1016/j.foodres.2012.10.004
Benítez-Cabello, A., Torres-Maravilla, E., Bermúdez-Humarán, L., Langella, P., Martín, R., Jiménez-Díaz, R., Arroyo-López, F. N., 2020. Probiotic Properties of Lactobacillus strains isolated from Table Olive Biofilms. Probiotics and Antimicrob. Proteins, 12(3), 1071-1082. https://doi.org/10.1007/s12602-019-09604-y
Biswas et al., 2016. CRISPR Detect: a flexible algorithm to define CRISPR arrays. BMC Genom. 17, 356. https://doi.org/10.1186/s12864-016-2627-0
Blin et al.., 2021. antiSMASH 6.0: improving cluster detection and comparison capabilities. Nucleic Acids Res. 49(W1), W29-W35. https://doi.org/10.1093/nar/gkab335
Bortolaia et al., 2020. ResFinder 4.0 for predictions of phenotypes from genotypes. J. Antimicrob. Chemother. 75, 3491-3500. https://doi.org/10.1093/jac/dkaa345
Camacho et al., 2009. BLAST+: architecture and applications. BMC Bioinform. 10(1), 421. https://doi.org/10.1186/1471-2105-10-421
Chen, Y. S., Wang, Y. C, Chow, Y. S., Yanagida, F., Liao, C. C., Chiu, C. M., 2014. Purification and characterization of plantaricin Y, a novel bacteriocin produced by Lactobacillus plantarum 510. Arch. Microbiol. 196(3), 193-9. https://doi.org/10.1007/s00203-014-0958-2
Conesa et al., 2005. Blast2GO: a universal tool for annotation, visualization and analysis in functional genomics research. Bioinform. 21, 3674-3676. https://doi.org/10.1093/bioinformatics/bti610
Crowley et al., 2013. Current perspectives on antifungal lactic acid bacteria as natural bio-preservatives. Trends Food Sci. Technol. 33, 93-109. https://doi.org/10.1016/j.tifs.2013.07.004
Elbehiry et al., 2022. Pseudomonas species prevalence, protein analysis, and antibiotic resistance: an evolving public health challenge. AMB Expr 12, 53. https://doi.org/10.1186/s13568-022-01390-1
Kaas et. al., 2014. Solving the Problem of Comparing Whole Bacterial Genomes across Different Sequencing Platforms. PLoS ONE 9(8), e104984. https://doi.org/10.1371/journal.pone.0104984
Kanehisa et al.., 2016. BlastKOALA and GhostKOALA: KEGG tools for functional characterization of genome and metagenome sequences. J. Mol. Biol. 428, 726-731. https://doi.org/10.1016/j.jmb.2015.11.006
Kessi et al.1999. Reduction of selenite and detoxification of elemental selenium by the phototrophic bacterium Rhodospirillum rubrum. Appl. Environ. Microbiol. 65, 4734-4740. https://doi.org/10.1128/AEM.65.11.4734-4740.1999
Leekitcharoenphon et al., 2014. Evaluation of Whole Genome Sequencing for Outbreak Detection of Salmonella enterica. PLoS ONE 9(2), e87991. https://doi.org/10.1371/journal.pone.0087991
Liu et al., 2019. VFDB 2019: a comparative pathogenomic platform with an interactive web interface. Nucleic Acids Res. 47, D687-D692. https://doi.org/10.1093/nar/gky1080
Martínez et al., 2020. Biotransformation of Selenium by Lactic Acid Bacteria: Formation of Seleno-Nanoparticles and Seleno-Amino Acids. Front. Bioeng. Biotechnol. 8, 506. https://doi.org/10.3389/fbioe.2020.00506
Mohamad et al., 2018. Evaluation of the antimicrobial activity of endophytic bacterial populations from chinese traditional medicinal plant licorice and characterization of the bioactive secondary metabolites produced by Bacillus atrophaeus against Verticillium dahliae. Front. Microbiol. 9, 924. https://doi.org/10.3389/fmicb.2018.00924
Mora-Villalobos et al., 2020. Multi-Product Lactic Acid Bacteria Fermentations: A Review. Ferment. 6, 23. https://doi.org/10.3390/fermentation6010023
Paysan-Lafosse, et al., 2022 InterPro in 2022 Nucleic Acids Research, Nov, (doi: 10.1093/nar/gkac993
Pérez-Díaz, et al., 2021. Genotypic and phenotypic diversity among Lactobacillus plantarum and Lactobacillus pentosus isolated from industrial scale cucumber fermentations. Food Microbiol. 94, 103652. https://doi.org/10.1016/j.fm.2020.103652
Pérez Montoro, et al., 2016. Fermented Aloreña table olives as a source of potential probiotic Lactobacillus pentosus strains. Front. Microbiol. 7, 1583. https://doi.org/10.3389/fmicb.2016.01583
Qian at al., 2003, The ND-Tree: A Dynamic Indexing Technique for Multidimensional Non-ordered Discrete Data Spaces, Proceedings 2003 VLDB Conference, Morgan Kaufmann, 620-631,https://doi.org/10.1016/B978-012722442-8/50061-6
Qiao et al. 2023. Selenite Bioremediation by Food-Grade Probiotic Lactobacillus casei ATCC 393: Insights from Proteomics Analysis. Microbiol. Spectr. e00659-23. https://doi.org/10.1128/spectrum.00659-23
Stamatakis, A., 2014. RAxML version 8: a tool for phylogenetic analysis and post-analysis of large phylogenies. Bioinform. 30(9), 1312-3. https://doi.org/10.1093/bioinformatics/btu033
Sun et al., 2015. Expanding the biotechnology potential of lactobacilli through comparative genomics of 213 strains and associated genera. Nat. Commun. 6, 8322. https://doi.org/10.1038/ncomms9322
Tenea, G. N., Ascanta, P., 2022. Bioprospecting of Ribosomally Synthesized and Post-translationally Modified Peptides Through Genome Characterization of a Novel Probiotic Lactiplantibacillus plantarum UTNGt21A Strain: A Promising Natural Antimicrobials Factory. Front. Microbiol. 13, 868025. https://doi.org/10.3389/fmicb.2022.868025
Tóth et al., 2021. Mobile Antimicrobial Resistance Genes in Probiotics. Antibiotics 10(11), 1287. https://doi.org/10.3390/antibiotics10111287
van Heel et al., 2018. BAGEL4: a user-friendly web server to thoroughly mine RiPPs and bacteriocins. Nucleic Acids Res. 46(W1), W278-W281. https://doi.org/10.1093/nar/gky383
Wu et al., 2011. WebMGA: a customizable web server for fast metagenomic sequence analysis. BMC Genom. 12, 444. https://doi.org/10.1186/1471-2164-12-444
Zankari et al., 2020. PointFinder: a novel web tool for WGS-based detection of antimicrobial resistance associated with chromosomal point mutations in bacterial pathogens. J. Antimicrob. Chemother. 72(10), 2764-2768. https://doi.org/10.1093/jac/dkx217
Zhang et al., 2004. A greedy algorithm for aligning DNA sequences. J. Comput. Biol. 7, 203-214. https://doi.org/10.1089/10665270050081478
Zhou et al., 2011. PHAST: a fast phage search tool. Nucleic Acids Res. 39, W347-W352. https://doi.org/10.1093/nar/gkr485

## Claims

1. *Lactiplantibacillus pentosus* strain CF-6HA deposited with the Spanish Type Culture Collection under accession number CECT 30896.

2. Pure bacterial culture or supernatant isolated from the strain of claim 1.

3. Composition comprising the culture or supernatant according to claim 2.

4. Non-therapeutic use of the strain according to claim 1, the pure bacterial culture according to claim 2, and/or the composition comprising the culture according to claim 3 as a probiotic for plants.

5. Non-therapeutic use of the strain according to claim 1, the pure bacterial culture according to claim 2 and/or the composition comprising the culture according to claim 3 as an antimicrobial agent against *Pseudomonas fragi.*

6. Non-therapeutic use according to the preceding claim, **characterised in that** it also acts simultaneously against one or more of the microorganisms selected from the list consisting of: *Enterococcus faecalis, Bacillus cereus, Staphylococcus aureus, Listeria innoccua, Escherichia coli, Salmonella enterica, Candida albicans, Pseudomonas syringae* and *Verticillium dahliae.*

7. Non-therapeutic use of the isolated supernatant according to claim 2 and/or of the composition comprising the supernatant according to claim 3 as an antimicrobial compound or composition against *Pseudomonas syringae, Verticillium dahliae, Pseudomonas fragi, Staphylococcus aureus, Bacillus cereus, Escherichia coli* and/or *Candida albicans*.

8. A strain according to claim 1, the pure bacterial culture according to claim 2, and/or the composition comprising the culture according to claim 3 for use as a probiotic medicament for humans and non-human mammals.

9. Strain according to claim 1, the pure bacterial culture according to claim 2 and/or the composition comprising the culture according to claim 3 for use as an antimicrobial medicament against *Pseudomonas fragi.*

10. Strain, culture and/or composition for use according to the preceding claim, **characterised in that** it is also used simultaneously as an antimicrobial medicament against one or more of the microorganisms selected from the list consisting of: *Enterococcus faecalis, Bacillus cereus, Staphylococcus aureus, Listeria innoccua, Escherichia coli, Salmonella enterica, Candida albicans, Pseudomonas syringae* and *Verticillium dahliae.*

11. An isolated supernatant according to claim 2 and/or the composition comprising the supernatant according to claim 3 for use as an antimicrobial medicament against *Pseudomonas syringae, Verticillium dahliae, Pseudomonas fragi, Staphylococcus aureus, Bacillus cereus, Escherichia coli* and/ or *Candida albicans*.

12. Use of strain according to claim 1 for obtaining pediocin and Plantaricin Y.

13. Use of strain according to claim 1 for bioremediation of media contaminated with selenium.

14. Use of strain according to claim 1 for the bioenrichment of foods fermented with selenium.
